# EUROPEAN PATENT APPLICATION

(11) **EP 4 732 852 A1**
(43) Date of publication of application: **29.04.2026**
(21) Application number: 25710517.1
(22) Date of filing: 30.01.2025
(51) Int. Cl.: A61L 2/07, A61L 11/00, B09B 3/45

(54) **SYSTEM FOR SOLID WASTE TREATMENT BY THERMAL HYDROLYSIS AND METHOD FOR SOLID WASTE TREATMENT THAT USES SAID SYSTEM**

(30) Priority: 31.01.2024 ES 202430082
(71) Applicant: Compsa, S.L., 28009 Madrid (ES)
(72) Inventor: PACHECO PERONA, Jesús, 28009 Madrid Madrid (ES)
(74) Representative: Ungria López, Javier
(86) International application number: PCT/ES2025/070040
(87) International publication number: WO 2025/163229

(57) **Abstract**

The present specification discloses an autoclave assembly, a thermal hydrolysis solid waste treatment system comprising at least two autoclave assemblies, and a thermal hydrolysis solid waste treatment method by means of the thermal hydrolysis solid waste treatment system. The present invention solves all the problems exhibited in the treatment of "complex" solid waste (composition of fibrous materials such as textiles, plastics), with advantages such as: being able to use a single inlet/outlet point of the treatment vessel located at the bottom portion, which saves on infrastructure; taking advantage of the storage capacity of the vertical arrangement; using conveyor systems that allow good fluid dynamics without clogging, which prevents the entanglement of fibrous materials; and using a system designed to move all the solid waste accumulated in the vertical vessel, which allows an internal recirculation during treatment that substantially improves heat transfer.

## Description

### TECHNICAL FIELD OF THE INVENTION

The present invention belongs to the technical field of solid waste treatment at the industrial level.

More specifically, the present invention relates to an autoclave assembly, to a solid waste treatment system using a thermal hydrolysis process, which has at least two autoclave assemblies, as well as to a solid waste treatment method using the system.

### BACKGROUND OF THE INVENTION

A number of industrial solid waste treatment systems and methods are known, such as, for example, those disclosed by European patent EP2519362, which belongs to the applicant. Said document discloses a solid waste treatment system comprising a reactor, or autoclave, equipped with an external vessel configured to hold solid waste under pressure, inside which there is arranged a reverse rotor comprising two cylindrical tubes coaxial to one another, fitted with helical blades rotating in reverse directions. In some embodiments of the invention, the treatment system, in addition to the reactor, may also comprise other autoclaves, acting as an inlet chamber, transit chamber or outlet chamber, which are also arranged horizontally and equipped with an internal screw conveyor. Furthermore, said autoclaves are positioned on top of each other by means of a vertical support structure.

Additionally, the autoclave assembly for the sterilisation of medical waste and the method of operation thereof are known in the prior art and are disclosed in PCT patent application with publication number WO2013/050822A1. The autoclave assembly disclosed in said PCT has the waste inlet at the top portion of the autoclave assembly. This is a limitation in terms of the height of the autoclave assembly, as the higher the autoclave assembly, the more energy is required to lift the medical waste to the top portion of the autoclave assembly. Furthermore, the autoclave assembly disclosed in the PCT agitates the waste by means of the rotation of an auger having arms or rods with scrapers fixed to them and distributed along the length of the auger to agitate all the material stored in the sterilisation chamber. This system would not be valid for treating complex waste such as MSW (Municipal Solid Waste) or other waste containing in its composition fibrous materials such as textiles, plastics, etc., which would become entangled on the arms and scrapers, causing an excessive increase in the electrical consumption of the geared motor "M" and stopping the auger. On the other hand, slow agitation of a complex solid waste does not greatly improve the heat transfer of the steam condensing on the solid waste.

There is a need in the sector to increase the performance of solid waste treatment systems and methods, their treated water and steam consumption. In addition, it would also be desirable to reduce the space required in the plant for its installation and reduce the complexity of the system, resulting in a lower probability of malfunctions and less need for investment in making a more robust system.

### TECHNICAL PROBLEM TO BE SOLVED

The present invention, unlike prior art solutions, solves the technical problem of how to treat municipal solid waste "MSW" containing in its composition fibrous materials such as textiles, plastics, etc., as they would become entangled at the lower outlet of the vertical autoclave assembly. For example, in the PCT document with publication number WO2013/050822A1 cited in the prior art section, municipal solid waste containing in its composition fibrous materials such as textiles, plastics, would become entangled with the support "25" and on the bearing "8a", blocking the passage of material.

The present invention solves all the problems presented by the treatment of "complex" solid waste (composition of fibrous materials such as textiles, plastics, etc.) such as MSW (Municipal Solid Waste), having the following advantages:
- Storage in a vertical vessel with loading/unloading of waste through the same opening at the bottom portion. This reduces the footprint of the installation and cost of conveyors required (reduces CAPEX).
- Treatment in vertical vessels also allows for a much higher utilisation of storage capacity compared to the horizontal autoclave system. This also results in reducing the footprint and CAPEX.
- Screw conveyor system designed for good fluid dynamics without clogging as the ends are free without any type of shaft support that would cause the entanglement of the fibrous materials.
- Vertical screw conveyor with blades specially designed to move all the solid waste accumulated in a vertical vessel, allowing, on the one hand, its internal recirculation during the treatment process and, on the other hand, its complete discharge after treatment. The internal recirculation allows the solid waste to precipitate in the form of a cascade once it reaches the upper portion of the screw conveyor, which greatly improves heat transfer phenomena. In addition, these blades also have the advantage of applying a shearing effect on the soft material such as organic matter, which reduces its particle size, improving the thermal hydrolysis treatment.

### DESCRIPTION OF THE INVENTION

The present specification discloses an autoclave assembly, a thermal hydrolysis solid waste treatment system comprising at least two autoclave assemblies, and a thermal hydrolysis solid waste treatment method by means of the thermal hydrolysis solid waste treatment system according to a first, a second and a third aspect of the invention, respectively. Throughout the present specification, the terms "pre-treatment material", "solid waste" and "municipal solid waste" have the same meaning: solid waste to be treated which may contain in its composition fibrous materials such as textiles, plastics, etc.

The present invention is intended to address all of the above problems.

In a first aspect of the invention, an autoclave assembly for thermal hydrolysis solid waste treatment is disclosed, the autoclave assembly comprising:
- a vertical vessel, having a truncated cone bottom portion, which is configured to hold a solid waste under pressure; wherein the vertical vessel comprises:
   ∘ a first common solid waste inlet/outlet area located at the bottom portion of the vertical vessel below the bottom portion of the vertical vessel;
   ∘ a lower treated solid waste discharge area;
   ∘ a steam inlet and a steam outlet located at the top portion of the vertical vessel, configured to pressurise and depressurise the vertical vessel with pressurised steam from a thermal hydrolysis process;
- a first horizontal conveyor comprising a solid waste inlet and a horizontal auger, which takes solid waste from the solid waste inlet of the first horizontal conveyor to the solid waste inlet/outlet of the vertical vessel;
- a second vertical conveyor comprising a vertical auger inside the vertical vessel configured to convey the solid waste by means of a downward flow and an upward flow; wherein the downward flow discharges the solid waste; wherein the vertical auger has a shaft and helical blades along the entire length of the shaft, wherein the helical blades have a diameter smaller than the diameter of the vertical vessel and comprises, at the height of the truncated cone bottom portion, sweeping blades the diameter of which extends to the inner perimeter of the vertical vessel without contacting same in such a way as to allow all the solid waste stored in the vertical vessel to be moved so that, depending on the direction of rotation of the geared motor to which the vertical auger is attached, a rotation of the vertical auger causing downward flow allows the treated solid waste to be discharged, while a rotation of the auger causing upward flow allows the solid waste to be fed and recirculated internally, facilitating its treatment by falling by gravity from the upper end of the vertical auger since, as it breaks up in the form of a "rain" or "cascade", it increases the surface area in contact with the process steam, resulting in a considerable improvement in heat transfer phenomena.

The design of the sweeping blades, the diameter of which extends to the inner perimeter of the vertical vessel without contacting same, prevents compaction of the solid waste and ensures that all the solid waste circulates through the vertical auger due to the thrust generated by the blades on the solid waste from the periphery of the vertical vessel (i.e. from the area closest to the inner surface of the vertical vessel) to the shaft of the vertical auger.

This thrust generated by the blades, as mentioned above, also generates an additional effect and benefits for the process. This effect is the shearing that occurs on the target materials, such as organic matter, tearing fibres and thus reducing the particle size of the material being treated. This increases the contact surface of the solid waste with the direct steam, improving the thermal hydrolysis treatment.

In one embodiment of the invention, the vertical vessel further comprises an auxiliary feed inlet at the top portion of the vertical vessel. It is thereby possible to supply a new load to the vertical vessel by means of a solid waste conveyor.

In another embodiment of the invention, the helical blades of the vertical auger comprise a helical rim around the perimeter, which, once the level of solid waste stored inside the vertical vessel is exceeded, helps to retain the solid waste that rises to the upper end of the vertical auger, thus maximising the flow falling in the form of a cascade and, therefore, the heat transfer from the steam to the solid waste. That is, the solid waste conveyed by the vertical auger is confined by the waste itself stored inside the vertical vessel (the auger has a drill effect) but may require the rim on the helical blades to retain it above said level of stored waste to maximise the flow that precipitates from the upper end of the vertical auger.

In another embodiment of the invention, the autoclave assembly further comprises at least one cutter arranged on the inner surface of the upper end of the vertical vessel and intended to cut and dislodge any fibrous material that may become entangled during rotation.

In another embodiment of the invention, the first horizontal conveyor and the second vertical conveyor comprise two motors supported by two driving frames, wherein the respective motors are attached to the horizontal auger and to the vertical auger, respectively, thereby causing rotational movement of the horizontal auger and of the vertical auger. However, the horizontal auger only works in the direction of rotation to feed untreated solid waste into the pressure vessel.

In another embodiment of the invention, the vertical auger has a low rotational speed. In particular, the rotational speed of the vertical auger is comprised between 5 and 30 rpm.

In another embodiment of the invention, the steam outlet of the vertical vessel is connected to a vacuum system configured to depressurise the autoclave assembly before opening same to the atmosphere for feeding or discharging treated solid waste.

In another embodiment of the invention, the steam inlet of the vertical vessel is connected to a steam generator producing pressurised steam.

In another embodiment of the invention, the steam inlet and outlet are through the same connection to the vertical vessel with the interposition of a self-cleaning filter.

In another embodiment of the invention, the solid waste inlet/outlet of the vertical vessel has stops that guide the vertical auger while keeping the lower tip of the shaft of the auger free, i.e. without any bearing or supported bushing.

The horizontal conveyor for feeding solid waste into the vertical vessel has a shaftless auger which, at its end opposite the geared motor, rests on its own sleeve. That is, like the lower end of the vertical auger, the horizontal auger is also unsupported, which prevents any entanglement of materials and greatly improves the fluid dynamics.

In a second aspect of the invention, the thermal hydrolysis solid waste treatment system is disclosed. The system is fitted with at least two autoclave assemblies, both with vertical vessels. Thanks to its particular configuration and the elements that make up same, it is possible to reduce the number of cycles necessary for correct treatment, which translates into a lower steam consumption and a longer useful life of the mechanical elements. Furthermore, the system according to the invention allows the effective residence time of the solid waste to be increased with respect to the already known systems, and is able to vary said residence time, depending on the specific needs of the waste to be treated. Furthermore, as the treatment system according to the present invention uses two interconnected and synchronised assemblies, it reduces steam consumption by recovering the depressurisation steam by heating a new batch of waste in the second autoclave assembly.

A further advantage is that it allows not only a further reduction of steam consumption, but also reduces the treated water and waste water generated during thermal hydrolysis coming from condensates, as compared to prior art treatment systems. Note that this only occurs if this residual steam is used to preheat a new batch of waste, otherwise the steam is condensed and stored for treatment.

Therefore, the thermal hydrolysis solid waste treatment system comprises at least a first autoclave assembly and a second autoclave assembly, both autoclave assemblies being as defined in any one of the embodiments of the first aspect of the invention. The steam inlet/outlet of the vertical vessel of the first autoclave is connected to the steam inlet/outlet of the vertical vessel of the second autoclave and to the vacuum system by means of valves, one for each steam inlet/outlet, and a third valve before the vacuum system. By means of this valve assembly, it is possible both to control the recovery of the steam that passes, by pressure difference, from one pressurised autoclave assembly to the other one that is at atmospheric pressure with a new batch of solid waste, and finally to depressurise the autoclave assembly completely by extracting the mixture of residual steam and non-condensables by means of the vacuum system.

As part of the system, various control and monitoring devices are provided, including level sensors to control the filling/emptying level by means of starting or stopping the geared motor of the horizontal feed conveyor, a pressure sensor to control the pressure by means of regulating the corresponding steam inlet/outlet valves, and a temperature sensor to monitor the treatment temperature.

This allows for there to be a fully automated process by means of controlling different parameters, resulting in increased safety and lower operating costs by minimising user interaction. For example, the entry and exit of solid waste is thereby fully automated. This ensures that the necessary safety conditions are in place before the vessel is accessible, and that the process parameters are suitable for this loading/unloading of the assembly.

Since the thermal hydrolysis process carried out inside the assembly works with high pressure and temperature parameters, it is extremely important to be able to check that these parameters are suitable before opening the assembly. In particular, pressure is an extremely important parameter to take into account from a safety point of view, given that, when proceeding with the opening cycle, failure to have a suitable magnitude could result in significant risks to the safety and health of the operators. For example, if this pressure were too high, when the tank is opened, it could cause a violent and uncontrolled discharge of pressure, endangering the safety of operators working in the vicinity of the assembly. Furthermore, if this were to take place at a high temperature it would pose additional risks due to the heat released.

Moreover, it should also be noted that from the point of view of the operation of the device, having a monitoring of certain process parameters and the degree of automation described also improves performance by being able to act on the process such that optimum values are maintained throughout the treatment of solid waste by means of thermal hydrolysis.

The third aspect of the invention discloses a thermal hydrolysis solid waste treatment method, using the thermal hydrolysis solid waste treatment system of the second aspect of the invention. The method comprises the following steps, having a predetermined duration:
a) loading into the first autoclave assembly a new batch of solid waste through the solid waste inlet port of the first horizontal conveyor and actuating the horizontal auger and the vertical auger until the solid waste reaches, preferably and at most, between 70% and 90% of the volume of the vertical vessel; in this step, the steam inlet and steam outlet valves are closed, and the treated solid waste outlet valve is also closed; the horizontal auger is stopped by stopping the geared motor of the horizontal auger;
b) compensating the pressure in the first autoclave assembly with a second autoclave assembly which is in step d), for which purpose: both autoclave assemblies are communicated by opening the corresponding residual steam inlet/outlet valves, keeping the vertical auger rotating in the direction of rotation to create an upward solid waste flow, increasing the heat exchange surface with the residual steam due to the cascade effect occurring when the solid waste falls from the top portion of the vertical auger; a residual steam flow from the second autoclave assembly to the first autoclave assembly is thereby created, due to differential pressure, to finally depressurise the second autoclave assembly almost completely, while in the first autoclave assembly virtually all the steam is utilised to preheat the solid waste recently fed in step a); in this step, the steam inlet valve is closed and the steam outlet valve is open; the treated solid waste outlet valve and the pre-treatment material inlet valve are closed;
c) pressurising the first autoclave assembly at the treatment pressure by means of injecting steam from the steam generator, which entails heating the solid waste at the temperature corresponding to the saturated steam pressure; in a first sub-step, the first autoclave assembly is pressurised and the solid waste is heated; in a second sub-step of this step "c)", conditions are maintained to give the predefined residence time; the operation of the vertical auger of the first autoclave assembly, with clockwise motor rotation for upward flow, allows a recirculation of the solid waste to be created by increasing the heat transfer surface with the residual steam due to the cascade effect occurring when the solid waste falls from the top portion of the vertical auger; all the solid waste is thereby quickly brought to its treatment temperature, while at the same time a mechanical effect that breaks down the organic matter without breaking the heavy foreign substances occurs; in this step, the steam outlet valve, the treated solid waste outlet valve and the solid waste inlet valve are kept closed, and the steam inlet valve is open;
d) compensating the pressure with the second autoclave assembly which, at this time, would be in step "a)" with a new batch of solid waste loaded and at atmospheric pressure. The operation of the vertical auger of the first autoclave assembly, with clockwise motor rotation for upward flow, allows the solid waste to be recirculated, increasing the flashing (release of steam from a superheated liquid or wet solid when the pressure of the process atmosphere it is in is reduced) due to the cascade effect occurring when the solid waste falls from the top portion of the vertical auger; a residual steam flow from the first autoclave assembly to the second autoclave assembly is thereby created, due to differential pressure, to finally depressurise the first autoclave assembly almost completely, while in the second autoclave assembly virtually all the steam is utilised to preheat the solid waste recently fed in step "a)"; in this step, the steam inlet valves, the treated solid waste outlet valve and the solid waste inlet valve are kept closed and the steam outlet valve is open;
e) depressurising the first autoclave assembly which, after the previous step (step "d)"), was slightly pressurised due mainly to the presence of non-condensables which need to be removed by means of a vacuum system specifically designed for this purpose. The operation of the inner vertical auger of the first autoclave assembly, with clockwise motor rotation for upward flow, allows a recirculation of the solid waste to be created by increasing the flashing due to the cascade effect occurring when the material falls from the upper portion of the auger; a mixed flow of non-condensables and residual steam towards the vacuum system is thereby created, due to differential pressure, allowing the autoclave assembly to be completely depressurised to atmospheric pressure; in this step, the steam inlet valve, the treated solid waste outlet valve and the solid waste inlet valve are kept closed, and the steam outlet valve is open;
f) discharging the batch of treated solid waste to the discharge conveyor (not shown) which would be just below the treated solid waste outlet valve; unlike all previous steps, in this step the vertical auger of the first autoclave assembly is rotated in the direction of rotation that is suitable for causing a downward treated solid waste flow to the treated solid waste outlet valve located at the bottom portion of the lower cone of the first autoclave assembly.

Optionally, step "a)" further comprises extracting the gas contained in the autoclave assembly, especially the air that has entered with the loading of the new batch of waste, by means of suction through the vacuum system. The heat transfer in the subsequent steps (steps "b)" and "c)") of treatment with direct steam is thereby improved. For this purpose, the solid waste inlet and outlet valves would be closed, as would the steam inlet valve, while the gas outlet valve to the vacuum system would be open. With respect to the screw conveyors, the horizontal material feed screw would be stopped, while the vertical screw could be stopped or operating in the direction of rotation for upward solid waste flow, which allows recirculation by releasing the gases occluded between the solid waste particles.

### BRIEF DESCRIPTION OF THE FIGURES

To complete the description of the invention, and for the purpose of helping to make the features thereof more readily understandable, according to a preferred exemplary embodiment thereof, a set of drawings is included wherein, by way of illustration and not limitation, the following figures have been represented:
Figure 1 shows an autoclave assembly for thermal hydrolysis solid waste treatment according to an embodiment of the present invention.
Figure 2 shows an isometric view of the blades fixed to the vertical auger inside the vessel.
Figure 3 shows a plan view of the blades fixed to the vertical auger inside the vessel.
Figure 4 shows a thermal hydrolysis solid waste treatment system comprising two autoclave assemblies according to the present invention.
Figures 5-10 show the operation of the autoclave assembly for the thermal hydrolysis solid waste treatment method of the present invention.

### DESCRIPTION OF A PREFERRED EMBODIMENT OF THE INVENTION

- 1: 1'. - Autoclave assembly;
- 2: Autoclave assembly system;
- 3: Vertical vessel; 3' cylindrical top portion; 3" truncated cone bottom portion;
- 4: First common solid waste inlet/outlet area, located at the bottom portion of the vertical vessel; 4': guide stops;
- 5: Vertical auger (second vertical conveyor); 5': blades; 5": helical rim;
- 6: Sweeping blades;
- 7: Driving frame of the vertical auger support;
- 8: Vertical auger geared motor;
- 9: Cutter or cutters, provided on the inside surface of the upper end of the vertical vessel; the cutters may incorporate a baffle or baffles;
- 10: Self-cleaning basket filter (connected to clean steam inlet and residual steam outlet valves); 10': steam inlet; 10": steam outlet.
- 11: Auxiliary feed inlet
- 12: First horizontal conveyor; 12': First conveyor enclosure;
- 13: Shaftless horizontal auger (first conveyor);
- 14: Driving frame of the horizontal auger support;
- 15: Horizontal auger geared motor;
- 16: Treated solid waste outlet valve; 16': treated solid waste outlet;
- 17: Solid waste inlet valve; 17': solid waste inlet;
- 18: Steam boiler;
- 19: Vacuum system;
- 20: Solid waste;
- 21: Treated solid waste;
- 22: Steam inlet valves 22', 22";
- 23: Steam outlet valves 23', 23".

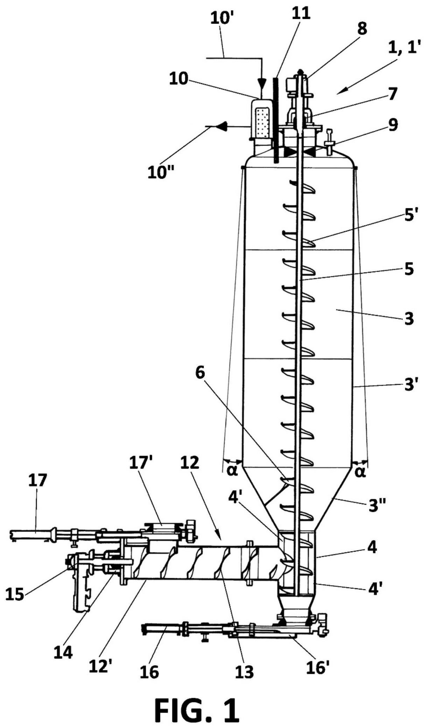

Figure 1 shows the autoclave assembly 1, 1' for thermal hydrolysis solid waste treatment according to an embodiment of the present invention. The autoclave assembly 1,1' comprises the circular vertical vessel 3. The circular vertical vessel 3 has a cylindrical or conical-shaped top portion 3' at an angle "α" with respect to the longitudinal axis of the vessel coinciding with the geometric axis of the cylindrical shape of the top portion 3'. Additionally, the circular vertical vessel 3 comprises a truncated cone bottom portion 3" that connects to the common solid waste inlet/outlet area 4 of the vertical vessel 3. This common solid waste inlet/outlet area 4 of the vertical vessel 3 is cylindrical in shape and has at its lower end a solid waste outlet 16', the opening and closing of which is actuated by the solid waste outlet valve 16. Additionally, the common solid waste inlet/outlet area 4 of the vertical vessel 3 has stops 4' guiding the vertical auger 5, keeping the lower end of the auger shaft 5 free, i.e. without any bearing or supported bushing. This prevents any obstruction (normally due to the entanglement of the solid waste at the end of the auger shaft) at the outlet of the vertical vessel 4 through the solid waste outlet valve.

The vertical vessel 3 has the vertical auger 5 that runs longitudinally through the vertical vessel 3, which is supported by the driving frame 7 and is moved by the geared motor 8. The driving frame 7 and the geared motor 8 are located above the top portion of the vertical vessel 3 to avoid contact with steam and solid waste from the process. To prevent fibers or other components of the solid waste from being becoming entangled at the top portion of the vertical conveyor shaft 5 at the top portion of the vertical vessel and inside same, one or more cutters 9 can be arranged (see Figure 1). Continuing with the top portion of the vertical vessel 3, the vertical vessel 3 comprises the filter 10 which in turn has the steam inlet 10' and the steam outlet 10". The filter 10 shown in Figure 1 is a self-cleaning basket filter since the clean steam flow used for the process is opposed to the dirty residual steam flow which is extracted after the thermal hydrolysis process is completed.

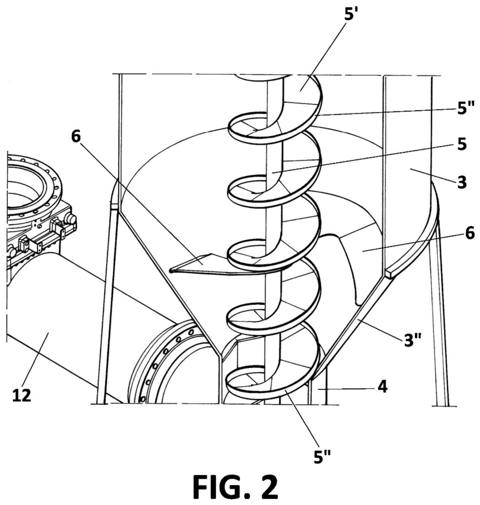

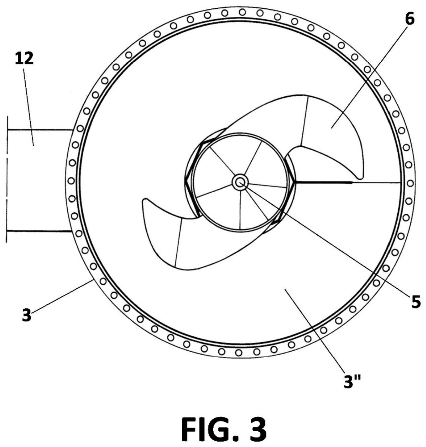

At the top portion of the vertical vessel 3 there is also an auxiliary feed inlet 11, which allows solid waste to be fed through the top portion of the vertical vessel 3. The vertical auger 5, at the height of the truncated cone bottom portion 3" of the vertical vessel 3, has the sweeping blades 6 (see Figures 1 to 3) which, together with a low rotational speed of the auger 5, has the technical effect of bringing the solid waste to the auger 5, which creates a recirculation of all the material stored in 3. The sweeping blades 6 have a diameter extending to the inner perimeter of the truncated cone bottom portion 3" of the vertical vessel 3, without contacting it (3"). The vertical auger 5 has the helical blades 5', which convey the solid waste to the upper end of the auger 5 or in the opposite direction of rotation, allowing the waste to be conveyed to the discharge. The assembly of the vertical auger 5, the vertical auger support driving frame 7 and the vertical auger geared motor 8, together with the elements of each of them, form the "second conveyor".

Continuing with Figure 1, the autoclave assembly 1,1' comprises the first conveyor 12, which has a cylindrical shape in a horizontal arrangement. The first conveyor 12 has the enclosure 12', which internally has the horizontal auger 13. The horizontal auger 13 has no shaft, being guided on the inside of the enclosure 12'. In addition, the auger 13 is driven by the horizontal auger geared motor 15, which is supported by the driving frame of the horizontal auger support 14 and attached to one end of the auger 13. Above the enclosure 12' and outside same, there is a solid waste inlet 17', the opening/closing of which is actuated by the solid waste inlet valve 17. The solid waste inlet 17' serves to receive the solid waste. The other end of the first conveyor 12 is connected to the inlet/outlet 4 of the vertical vessel 3. In this way, the solid waste that is introduced through the solid waste inlet valve 17 is moved to the first common solid waste inlet/outlet area 4 of the vertical vessel 3 without obstructions because the horizontal auger 13 has no shaft. This prevents obstructions due to entanglements that occur in conventional augers with a shaft.

The autoclave assembly 1,1' is airtight and can be pressurised to carry out a thermal hydrolysis solid waste treatment method.

The helical blades 5' may comprise a helical rim 5" on the perimeter which helps the solid waste conveyed by the blades 5' to reach the upper end of the vertical auger 5. The rim helps to ensure that the pre-treatment material (solid waste), which is conveyed upwards by the vertical auger, does not become dislodged above the height corresponding to the level of solid waste stored inside the vessel. Below this height, the solid waste inside the auger is confined by the solid waste stored along the periphery, and therefore vertical conveying of all the material is ensured.

As an alternative to the cutter(s) 9, a baffle (not shown) could also be arranged on the inner surface of the upper end of the vertical vessel 3, intended to dislodge and distribute the solid waste raised by the second vertical conveyor.

The vertical auger geared motor 8 can rotate the vertical auger 5 in both directions, respectively, while the horizontal auger geared motor 15 will rotate the horizontal auger 13 in the feed direction. The low rotational speed also reduces the power consumption of the vertical auger geared motor 8.

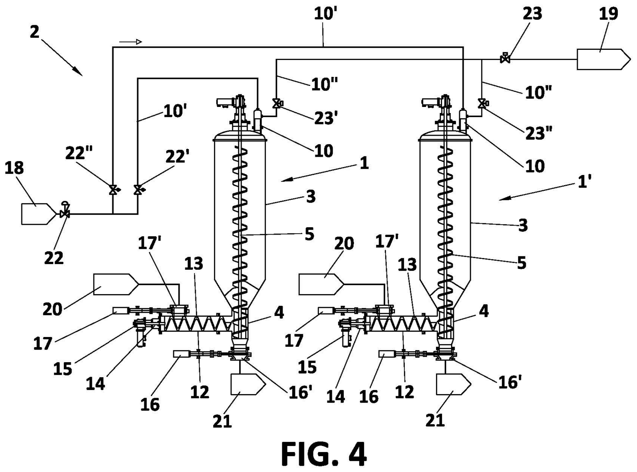

Figure 4 shows an exemplary embodiment of a thermal hydrolysis solid waste treatment system 2 according to the present invention. The system 2 comprises two autoclave assemblies 1 and 1' as described above in Figures 1 to 3. As can be seen in Figure 4, both autoclave assemblies 1 and 1' are connected to a steam generator 18, which generates pressurised steam that reaches the filters 10 of both autoclave assemblies 1, 1' through ducts 10'. The amount of steam circulating from the steam generator 18 to each of the autoclave assemblies 1, 1' is controlled by valves 22 (main), 22' (for autoclave assembly 1) and 22" (for autoclave assembly 1').

The residual steam outlet of the vertical vessel 3 is connected to a vacuum system 19 through ducts 10" intended to facilitate the depressurisation of the autoclave assembly 1, 1' before opening same to the atmosphere for feeding a new batch of solid waste 20 (solid waste - municipal solid waste) or discharging a batch of treated solid waste 21. The amount of residual steam circulating from each of the autoclave assemblies 1, 1' to the vacuum system 19 and the amount of residual steam circulating between autoclave assemblies 1, 1' is controlled by valves 23 (main), 23' (for autoclave assembly 1) and 23" (for autoclave assembly 1'). Advantageously, the entry and exit of steam takes place through the same connection to the vertical vessel 3, through the basket filter 10, which is thereby made to be self-cleaning by introducing the clean steam (coming from the steam generator 18) in the direction opposite to the residual steam exiting towards the vacuum system 19 or other autoclave assembly during depressurisation.

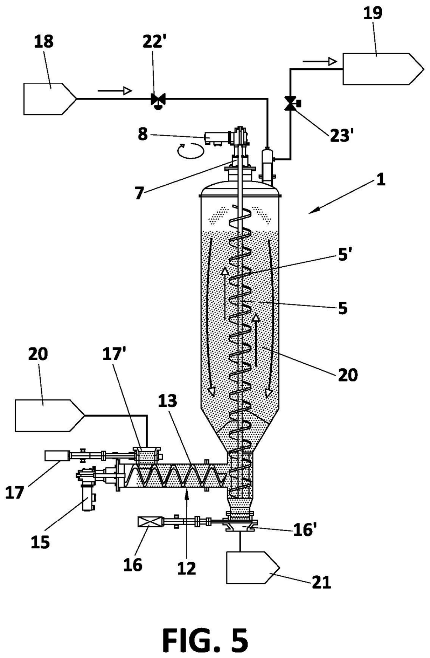

Figures 5 to 10 show the thermal hydrolysis solid waste treatment method of the present invention as applied to the thermal hydrolysis solid waste treatment system of Figure 4. The method comprises the following steps, having a predetermined duration:
Step 1: (Figure 5) loading into the autoclave assembly 1, a new batch of solid waste 20 through the solid waste inlet 17' of the first horizontal conveyor 12 and actuating the horizontal auger 13 and the vertical auger 5 until the solid waste 20 reaches, preferably and at most, between 70% and 90% of the volume of the vertical vessel (it is necessary to leave a free volume at the top portion to facilitate the heat transfer from the steam to the pre-treatment material); the horizontal auger 13 and vertical auger 5 are actuated by means of actuating the geared motor 8 and 15 ("on" state = depicted with white background), respectively; in this step, valves 22' and 23' are closed ("closed" state = depicted entirely in black), and outlet valve 16 ("closed" state = depicted with an "x" inside) and outlet 16' are also closed. In this step, the solid waste inlet valve 17 is also open ("open" state = depicted with white background).

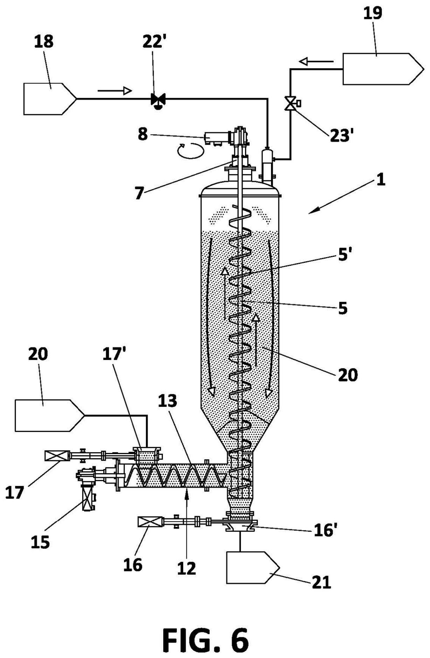

Optionally (FIG. 6), the gas contained in the autoclave assembly can be extracted, especially the air that has entered with the loading of the new batch of waste, by means of suction through the vacuum system. This improves the heat transfer in subsequent steps in which treatment with direct steam takes place. For this purpose, the solid waste inlet and outlet valves would be closed, as would the steam inlet valve, while the gas outlet valve to the vacuum system would be open. With respect to the conveyors, the horizontal material feed screw is stopped, while the vertical screw could be stopped or operating in the direction of rotation for upward solid waste flow, which allows recirculation by releasing the gases occluded between the solid waste particles.

Step 2: (Figure 6) compensating the pressure in the autoclave assembly 1 with the autoclave assembly 1' in step 4; i.e., both autoclave assemblies 1 are communicated by opening the residual steam inlet/outlet valve 23' ("open" state = depicted with a white background), keeping the vertical auger 5 rotating, by means of starting the geared motor 8, in the suitable direction of rotation to create an upward solid waste flow, increasing the heat exchange surface with the residual steam due to the cascade effect occurring when the solid waste falls from the top portion of the auger 5; a residual steam flow from autoclave assembly 1' to autoclave assembly 1 is thereby created, due to differential pressure, to finally depressurise autoclave assembly 1' almost completely, while in autoclave assembly 1 virtually all the steam is utilised to preheat the solid waste recently fed in step 1; by means of its geared motor 15, the horizontal screw conveyor 13 is stopped ("stopped " state = depicted with an "x" inside); in this step, the steam inlet valve 22' is closed; the treated solid waste outlet valve 16 (and the treated solid waste outlet 16'), the inlet valve 17 ("closed " state = depicted with an "x" inside) and the inlet 17' are closed.

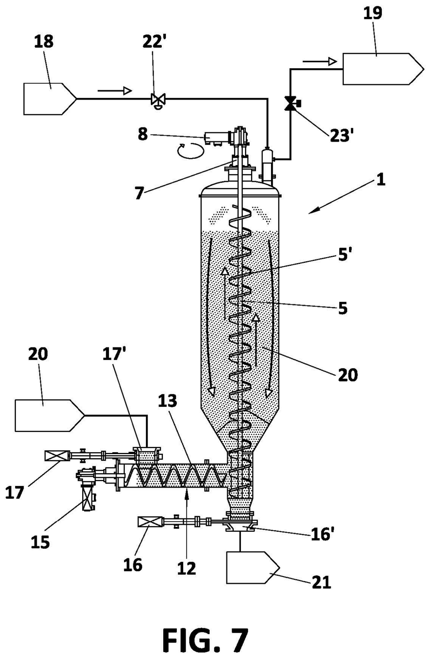

Step 3: (Figure 7) pressurising the autoclave assembly 1 at the treatment pressure by means of injecting steam from the steam generator, which entails, in a first sub-step, pressurising the autoclave assembly 1 and heating the solid waste at the temperature corresponding to the saturated steam pressure; in a second sub-step of this step 3, conditions are maintained to give the required residence time depending on the specific treatment requirements decided on in each project (more or less decomposition, sanitisation, sterilisation, etc.); the operation of the vertical screw conveyor 5 by means of starting the geared motor 8, inside the autoclave assembly 1, with motor rotation in the suitable direction to create an upward flow, allows the solid waste to be recirculated, increasing the heat transfer surface with the residual steam due to the cascade effect occurring when the solid waste falls from the top portion of the vertical auger 5; all the solid waste is thereby quickly brought to its treatment temperature, while at the same time a mechanical effect that breaks down the organic matter without breaking the heavy foreign substances occurs; it is therefore important to emphasise that, with this system, it is possible to keep the solid waste moving during the entire treatment time and, in turn, said treatment time can be adjusted according to the specific requirements of each project with the only variation being the production capacity; in this step, the valve 23' ("closed" state = depicted entirely in black), the treated solid waste outlet valve 16 and the treated solid waste outlet 16' are closed; and, the steam inlet valve 22' ("open" state = depicted with white background) is open. The geared motor 15 is stopped; and, the inlet valve 17 and the inlet 17' are closed.

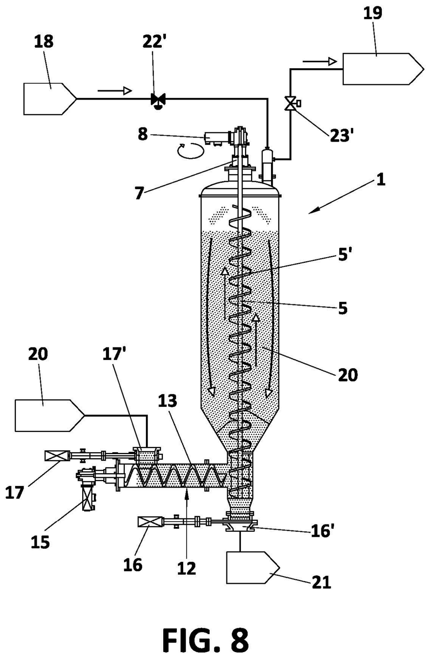

Step 4: (Figure 8) compensating the pressure with the autoclave assembly 1' which, at this time, would be in "Step 1" with a new batch of solid waste loaded and at atmospheric pressure. The operation of the vertical auger 5 by means of actuating the geared motor 8 of the autoclave assembly 1, with clockwise motor rotation for upward flow, allows the solid waste to be recirculated by increasing the flashing (release of steam from a superheated liquid or wet solid when the pressure of the process atmosphere it is in is reduced) due to the cascade effect occurring when the solid waste falls from the top portion of the vertical auger 5. A residual steam flow from autoclave assembly 1' to autoclave assembly 1 is thereby created, due to differential pressure, to finally depressurise autoclave assembly 1' almost completely, while in autoclave assembly 1 virtually all the steam is utilised to preheat the solid waste recently fed in step 1; in this step, the valve 22' ("closed" state = depicted entirely in black), treated solid waste outlet valve 16, the treated solid waste outlet 16', the solid waste inlet valve 17 and the solid waste inlet 17' are kept closed. The steam outlet valve 23' ("open" state = depicted with white background) is open. The geared motor 15 is stopped.

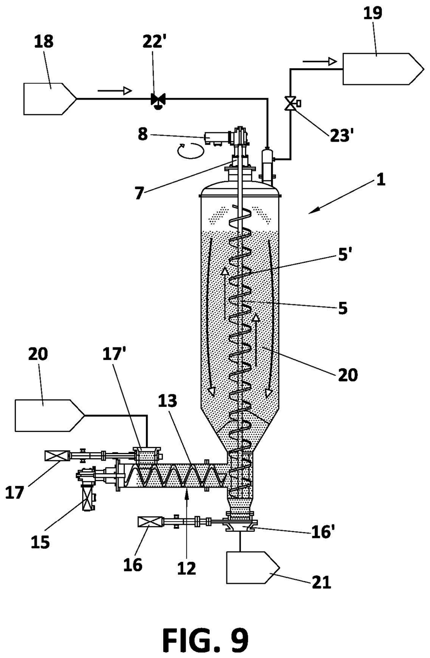

Step 5: (Figure 9) depressurising autoclave assembly 1 which, after the previous step (Step 4), was slightly pressurised due mainly to the presence of non-condensables which need to be removed by means of a vacuum system specifically designed for this purpose. The operation of the vertical auger 5 inside the autoclave assembly 1 by means of actuating the geared motor 8, with motor rotation in the suitable direction to create an upward flow, allows the solid waste to be recirculated, increasing the flashing due to the cascade effect occurring when the material falls from the top portion of the auger. A mixed flow of non-condensables and residual steam towards the vacuum system is thereby created, due to differential pressure, allowing the autoclave assembly to be completely depressurised to atmospheric pressure; in this step, the valve 22', the treated solid waste outlet valve 16 and the treated solid waste outlet 16' are kept closed. The steam outlet valve 23' is open. The geared motor 15 is stopped. The solid waste inlet valve 17 and the solid waste inlet 17' are closed.

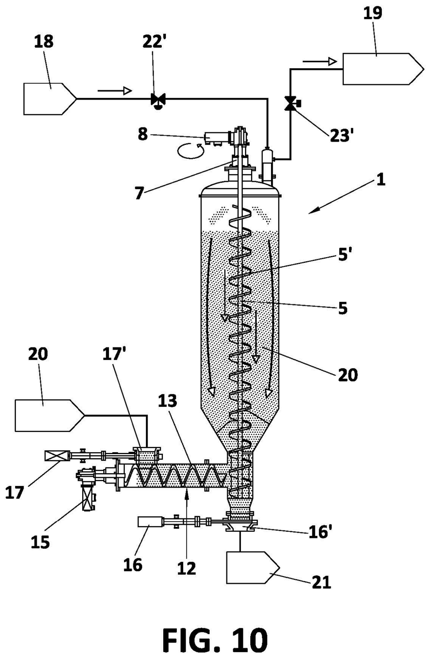

Step 6: (Figure 10) discharging the batch of treated solid waste to the discharge conveyor (not shown) which would be just below the treated solid waste outlet 16'. Unlike all the previous steps, in this step the vertical auger 5 of the autoclave assembly is rotated by means of starting the geared motor 8 in the opposite direction to create a downward treated solid waste flow towards the treated solid waste outlet 16' located below the first common solid waste inlet/outlet area 4. In this step, valves 22' and 23' ("closed" state = depicted entirely in black) are closed. The treated solid waste outlet valve 16 and the treated solid waste outlet 16' are open ("open" state = depicted with white background). The geared motor 15 is stopped. The solid waste inlet valve 17 and the solid waste inlet 17' are closed.

## Claims

1. An autoclave assembly for thermal hydrolysis solid waste treatment, the autoclave assembly (1,1') comprises:
- a vertical vessel (3), having a truncated cone bottom portion (3"), which is configured to hold a solid waste (20) under pressure; wherein the vertical vessel (3) comprises:
∘ a first common solid waste inlet/outlet area (4) located at the bottom portion of the vertical vessel (3) below the bottom portion (3") of the vertical vessel (3);
∘ a lower treated solid waste discharge area (16');
∘ a steam inlet (10') and a steam outlet (10") located at the top portion of the vertical vessel (3), configured to pressurise and depressurise the vertical vessel (3) with pressurised steam from a thermal hydrolysis process;
- a first horizontal conveyor (12,14,15) comprising a valve (17) to control the solid waste inlet and a horizontal auger (13), which takes solid waste (20) from the solid waste inlet of the first horizontal conveyor (12) to the solid waste inlet/outlet (4) of the vertical vessel;
- a second vertical conveyor (5,7,8) comprising a vertical auger (5) inside the vertical vessel (3) configured to convey the solid waste (20) by means of a downward flow and an upward flow; wherein the downward flow discharges the solid waste; wherein the vertical auger (5) has a shaft and helical blades (5') along the entire length of the shaft, wherein the helical blades (5') have a diameter smaller than the diameter of the vertical vessel (3) and comprises, at the height of the truncated cone bottom portion (3"), sweeping blades (6) the diameter of which extends to the inner perimeter of the vertical vessel (3) without contacting same in such a way as to allow the solid waste stored in the vertical vessel to be moved so that, depending on the direction of rotation of the geared motor to which the vertical auger is attached, a rotation of the vertical auger (5) causing downward flow allows the treated solid waste (21) to be discharged, while a rotation of the auger causing upward flow allows the solid waste (20) to be fed and recirculated internally, facilitating its treatment by falling by gravity from the upper end of the vertical auger (3), breaking down in the form of a "rain" or "cascade", increasing the surface area in contact with the process steam.

2. The autoclave assembly according to claim 1, wherein the helical blades (5') of the vertical auger (5) comprise a helical rim (5") on the perimeter.

3. The autoclave assembly according to claim 1, wherein the autoclave assembly (1,1') further comprises at least one cutter (9) arranged on the inner surface of the upper end of the vertical vessel (3).

4. The autoclave assembly according to claim 1, wherein the first horizontal conveyor and the second vertical conveyor comprise two motors (8, 15) supported by two driving frames (7, 14), wherein the respective motors (8, 15) are attached to the horizontal auger (13) and to the vertical auger (5), respectively, thereby causing rotational movement of the horizontal auger (13) in the direction of rotation of the feed and of the vertical auger (5) in both directions of rotation.

5. The autoclave assembly according to claim 4, wherein the vertical auger (5) has a low rotational speed, preferably between 5 and 30 rpm.

6. The autoclave assembly according to claim 1, wherein the steam outlet (10") of the vertical vessel (3) is connected to a vacuum system (19) configured to depressurise the autoclave assembly (1,1') before opening same to the atmosphere for feeding or discharging treated solid waste (21).

7. The autoclave assembly according to claim 1, wherein the steam inlet (10') of the vertical vessel (3) is connected to a steam generator (18) producing pressurised steam.

8. The autoclave assembly according to any one of the preceding claims, wherein the steam inlet (10') and outlet (10") are through the same connection to the vertical vessel with the interposition of a self-cleaning filter (10).

9. The autoclave assembly according to claim 1, wherein the vertical vessel (3) above (3') the bottom portion (3") of the vertical vessel (3), has a shape selected from a cylindrical and conical shape.

10. The autoclave assembly according to claim 1, wherein the solid waste inlet/outlet (4) of the vertical vessel has stops (4') guiding the vertical auger (5).

11. The autoclave assembly according to claim 1, wherein the vertical vessel (3) further comprises an auxiliary feed inlet (11) at the top portion of the vertical vessel (3).

12. A thermal hydrolysis solid waste treatment system, comprising at least a first autoclave assembly (1) and a second autoclave assembly (1'), both autoclave assemblies being as defined in any one of claims 1 to 11, wherein the steam inlet/outlet (10") of the vertical vessel (3) of the first autoclave is connected to the steam inlet/outlet (10") of the vertical vessel (3) of the second autoclave (1') and to the vacuum system (19) by means of valves (23',23"), one for each steam inlet/outlet, and a third valve (23) before the vacuum system (19), in such a way that the pressurised steam from one autoclave assembly is transferred, by pressure difference, to the other autoclave assembly, where it condenses by yielding its energy to preheat a solid waste.

13. A thermal hydrolysis solid waste treatment method, using the thermal hydrolysis solid waste treatment system of claim 12, the method comprising the following steps, having a predetermined duration:
a) loading into the first autoclave assembly (1) a new batch of solid waste (20) through the solid waste inlet port (17) of the first horizontal conveyor (12) and actuating the horizontal auger (13) and the vertical auger (5) until the solid waste (20) reaches, preferably and at most, between 70% and 90% of the volume of the vertical vessel (3);
b) compensating the pressure in the first autoclave assembly (1) with a second autoclave assembly (1') which is in step d), for which purpose: both autoclave assemblies (1,1') are communicated by opening the inlet/outlet valves (23', 23"), keeping the vertical auger (5) rotating in the direction of rotation suitable for creating an upward flow for the recirculation of the solid waste (20);
c) pressurising the first autoclave assembly (1) at the treatment pressure by means of injecting steam from the steam generator (18), which entails, in a first sub-step, pressurising the first autoclave assembly (1) and heating the solid waste (20) at the temperature corresponding to the saturated steam pressure; in a second sub-step of this step "c)", the conditions are maintained to give the predefined residence time;
d) compensating the pressure with the second autoclave assembly (1') which, at this time, would be in step "a)" with a new batch of solid waste loaded and at atmospheric pressure;
e) depressurising the autoclave assembly (1);
f) discharging the treated solid waste (21).

14. The thermal hydrolysis solid waste treatment method according to claim 13, **characterised in that** step "a)" further comprises extracting the gas contained in the autoclave assembly by means of suction through the vacuum system.
